# EUROPEAN PATENT SPECIFICATION

(11) **EP 1 905 780 B1**
(45) Date of publication and mention of the grant of the patent: **23.12.2009**
(21) Application number: 07014658.4
(22) Date of filing: 26.07.2007
(51) Int. Cl.: C07K 14/47, C12N 15/11, A61K 38/17, A61K 39/395, A61K 31/711, G01N 33/50

(54) **Cancer suppressing agent**
Krebsunterdrückender Wirkstoff
Agent d'élimination du cancer

(30) Priority: 27.07.2006 JP 2006204601
(43) Date of publication of application: 02.04.2008
(73) Proprietor: FUJIFILM Corporation, Minato-ku Tokyo 106-8620 (JP); Tokyo Medical and Dental University, Tokyo 113-8510 (JP)
(72) Inventor: Inazawa, Johji, Tokyo 113-8510 (JP); Imoto, Issei, Tokyo 113-8510 (JP); Saigusa, Kuniyasu, Tokyo 113-8510 (JP)
(74) Representative: HOFFMANN EITLE

(56) References cited:
- EP-A- 1 410 804
- SAIGUSA K ET AL: "RGC32, a novel p53-inducible gene, is located on centrosomes during mitosis and results in G2/M arrest" ONCOGENE, vol. 26, no. 8, February 2007 (2007-02), pages 1110-1121, XP002469883 ISSN: 0950-9232
- IMOTO ISSEI ET AL: "RGC32, a novel p53-inducible tumor-suppressor gene, is located on centrosomes during mitosis and results in G2/M arrest." PROCEEDINGS OF THE AMERICAN ASSOCIATION FOR CANCER RESEARCH ANNUAL MEETING, vol. 48, April 2007 (2007-04), page 604, XP001537854 & 98TH ANNUAL MEETING OF THE AMERICAN-ASSOCIATION-FOR-CANCER-RESEARCH; LOS ANGELES, CA, USA; APRIL 14 -18, 2007 ISSN: 0197-016X
- FOSBRINK M ET AL: "Overexpression of RGC-32 in colon cancer and other tumors" EXPERIMENTAL AND MOLECULAR PATHOLOGY, ACADEMIC PRESS, US, vol. 78, no. 2, April 2005 (2005-04), pages 116-122, XP004745202 ISSN: 0014-4800
- BADEA TUDOR C ET AL: "Molecular cloning and characterization of RGC-32, a novel gene induced by complement activation in oligodendrocytes" JOURNAL OF BIOLOGICAL CHEMISTRY, vol. 273, no. 41, 9 October 1998 (1998-10-09), pages 26977-26981, XP002469887 ISSN: 0021-9258
- INAZAWA JOHJI ET AL: "Comparative genomic hybridization (CGH)-arrays pave the way for identification of novel cancer-related genes." CANCER SCIENCE JUL 2004, vol. 95, no. 7, July 2004 (2004-07), pages 559-563, XP002469885 ISSN: 1347-9032
- SAIGUSA K ET AL: "Overexpressed Skp2 within 5p amplification detected by array-based comparative genomic hybridization in associated with poor prognosis of glioblastomas" CANCER SCIENCE 2005 UNITED KINGDOM, vol. 96, no. 10, 2005, pages 676-683, XP002469886 ISSN: 1347-9032

## Description

### Technical Field

The present invention relates to a cancer suppressor gene and medical uses of a protein encoded by such gene.

### Background Art

It is long known that cancer onset is due to mutation or quantitative alteration of proteins in cells. Recent developments in gene engineering have made it possible to analyze the amplification of genes encoding specific proteins or genetic mutation in cancer cells, resulting in striking advances in the field of cancer research. So-called oncogenes involved in carcinogenesis of cells or abnormal proliferation of cancer cells have been analyzed and identified to date. Meanwhile, cancer suppressor genes have been attracting a great deal of attention for several years because of their involvement in carcinogenesis through mutation or lowered expression thereof. Examples of cancer suppressing genes that have been discovered so far include Rb gene of retinoblastoma, p53 gene and APC gene of large bowel cancer, and WT1 gene of Wilms tumor. An example of a cancer suppressing agent using the WT1 gene has also been reported (WO2003/002142).

Involvement of not only one abnormal gene but also a plurality of abnormal genes in cancer development, cancer advancement, cancer malignant alteration, cancer metastasis, and the like has been gradually revealed. It is thought that more unidentified cancer gene and cancer suppressing genes exist. Many genes having an effect of suppressing cancer are known. In most cases, such genes have been selected by an approach (Yasuhide Yamashita, et al., World J Gastroenterol, 11(33): 5129-5135, 2005) that involves staining chromosomal DNA for visualization and discovering gene mutations in patients or a method that involves selecting a rough range of gene deletion via LOH (Loss Of Heterozygosity) analysis and then narrowing down critical gene regions (WO01/032859). However, these methods have drawbacks such that the number of detectable DNA deletion regions will be enormous, meaning that narrowing down of critical gene regions will require much time and effort. Hence, these conventional methods have limitations as measures for searching for cancer suppressing genes. Moreover, it has been difficult to conduct malignancy determination through the use of conventional separation-discrimination methods for pathological conditions of cancer using genes.

### Disclosure of the Invention

An object of the present invention is to provide an agent for suppressing cancer as defined in the claims containing a cancer suppressing gene that is discovered through the use of a novel method rather than through existing methods. Another object of the present invention is to provide an agent for suppressing cancer containing a protein that is encoded by the cancer suppressing gene. Also described is a method for diagnosing malignancy of the pathological conditions of a cancer patient through measurement of the amount of the cancer suppressing gene existing or the expression level of the cancer suppressing gene in the patient.

The present inventors have searched energetically for partial DNA deletion regions in glioma cases to achieve the above objects. Glioma is mainly classified into astrocytoma, oligodendroglioma, ependymoma, and glioblastoma which has the highest malignancy. Glioma accounts for 30% to 40% of all brain tumor cases. To specify a cancer-associated gene in glioma cases, the present inventors have screened for a gene the deletion of which has taken place at high frequency in cancer cases through the use of a newly developed array CGH method (Inazawa J., et al., Cancer Sci. 95(7), 559, 2004). Furthermore, the present inventors have identified an RGC32 gene (response gene to complement 32) through the combined use of a cDNA microarray and an RT-PCR method. This gene is deficient in the DNA of glioma cases and the expression thereof is significantly suppressed. Furthermore, the present inventors have confirmed that when cancer cells lacking the RGC32 protein are caused to express the RGC32 protein, both anchorage-dependent proliferation potency and cell proliferation rate are lowered. Hence, the present inventors have demonstrated that the RGC32 protein is capable of functioning as a cancer suppressing gene product. The present inventors have completed the present invention based on these findings.

Specifically, the present invention provides an agent for suppressing cancer which comprises an RGC32 gene or a gene homologous thereto.

Preferably, the gene or the gene homologous thereto is incorporated into a vector.

Preferably, the vector is a viral vector or a plasmid vector for expression in animal cells.

Preferably, the viral vector is a retrovirus vector, an adenovirus vector, an adeno-associated virus vector, a baculovirus vector, a vaccinia virus vector, or a lentivirus vector.

Preferably, the gene or the homologous gene is encapsulated in a liposome.

Another aspect of the present invention provides an agent for suppressing cancer which comprises an RGC32 protein or a protein homologous thereto.

Disclosed is also a method for diagnosing cancer which comprises the step of analyzing an RGC32 gene in a specimen sample using DNA or RNA containing the whole or a portion of the RGC32 gene.

Preferably, the analysis involves detecting gene mutation or an abnormal expression level of the gene.

Also described is a method for diagnosing cancer which comprises the step of analyzing an RGC32 protein in a specimen sample using an antibody against the RGC32 protein or a fragment thereof.

Preferably, the analysis involves detecting an abnormal expression level of the protein.

### Brief Description of the Drawings

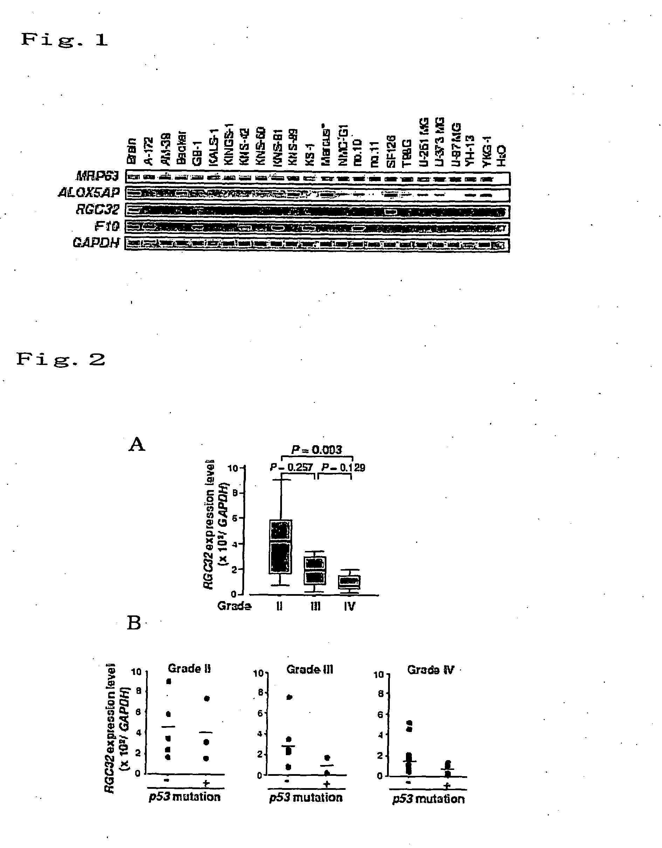

Fig. 1 shows the messenger RNA expression levels of MRP63, ALOX5AP, RGC32, and F10 genes in 22 types of glioma cell line and the normal brain, as detected by RT-PCR. Gene deletion on chromosome 13q was confirmed in the case of Marcus cells (indicated with asterisk) by array CGH analysis.
Fig. 2A shows a correlation between the messenger RNA expression levels of the RGC32 gene in 35 clinical glioma specimens and the grades determined based on the histopathological classification. The messenger RNA expression levels were measured by the real-time RT-PCR method using a GAPDH gene as a control. A significant inverse correlation was observed between grades (low-grade diffuse astrocytoma (Grade II) to glioblastoma multiforme (GBM, Grade IV)) and RGC32 gene expression levels. Fig. 2B shows the results of examining RGC32 gene expression levels in view of the presence or the absence of p53 gene point mutation for each grade determined based on the pathological classification. p53 gene point mutation was present regardless of Grade. Furthermore, RGC32 gene expression levels were observed to have a tendency to decrease in the cases with p53 gene point mutation, but there were no significant differences.

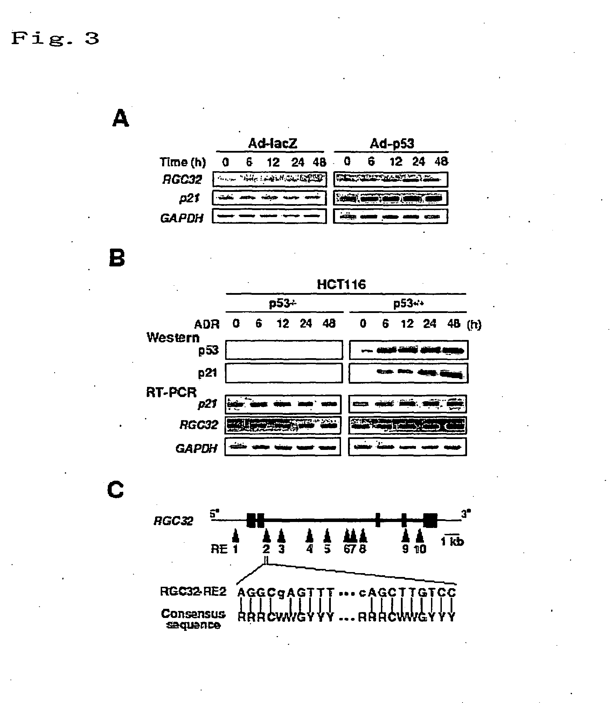

Fig. 3A shows the results of infecting U-373 MG cells observed not to express RGC32 gene messenger RNA with adenovirus Ad-p53 and control adenovirus Ad-lacZ and then performing RT-PCR analysis using p21 and GAPDH as a positive control. Fig. 3B shows p53 and p21 genes of HCT116 cells homozygously lacking p53 gene (p53-/-) and normal (p53+/+) HCT116 cells, as analyzed by Western blotting after addition of adriamycin (ADR) that induces DNA damage. Fig. 3B also shows the results of analyzing p21, RGC32, and control GAPDH genes by RT-PCR. Fig. 3C schematically shows the genomic DNA structure of the RGC32 gene. Black boxes represent exon structures. Wedge shapes represent p53 response sequences (consensus sequences). Comparison with an actual p53 response sequence in terms of RGC32-RE2 is also shown. Capital letters represent nucleotides corresponding to those in the p53 response sequence. Lowercase letters represent nucleotides not corresponding to the same. R represents a purine nucleotide, Y represents a pyrimidine nucleotide, and W represents A or T nucleotide.

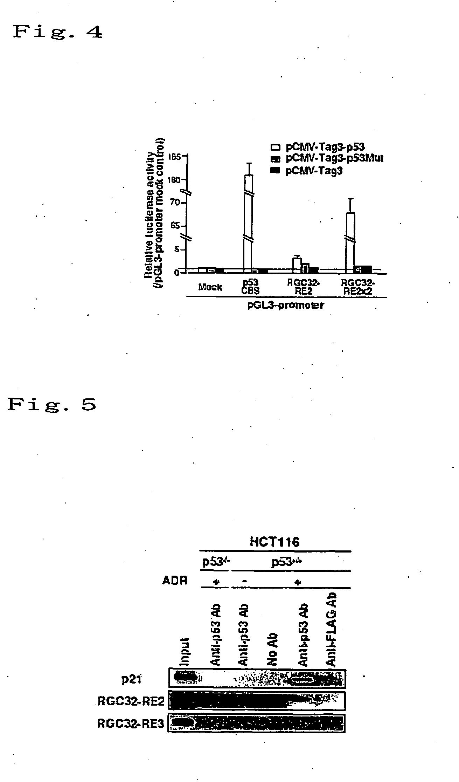

Fig. 4 shows the results of determining (3 times each) reporter activity by binding RGC32-RE2 and RGC32-RE2 × 2 (2 copies of RGC32-RE2) to luciferase reporter plasmids (pGL3-RGC32-RE2 and pGL3-RGC32-RE2 × 2), constructing the plasmids, and then transfecting SaOS2 cells with the plasmids, p21 (pGL3-p53CBS) as a positive control, a phRL-TK vector, and wild-type p53 (pCMV-Tag3-p53) or mutant p53 (pCMV-Tag3-p53Mut) having a minimum SV40 promoter or control (pCMV-Tag3) The longitudinal axis represents the relative activity of the pGL3 promoter to the Mock control.
Fig. 5 shows the results of preparing cell disruption solutions differing in terms of the presence or the absence of the addition ofADR (1 µg/ml, 24 hours) using HCT116 (p53-/-) or HCT116 (p53+/+) and detecting DNAs contained in precipitates (materials that had been obtained by subjecting the solutions to immunoprecipitation using an anti-p53 antibody or an anti-FLAG antibody as a negative control) by PCR using a p21 promoter region and primers specific to RGC32-RE2 and RGC32-RE3. "Input" represents the result of the positive control obtained using a cell disruption solution before immunoprecipitation.

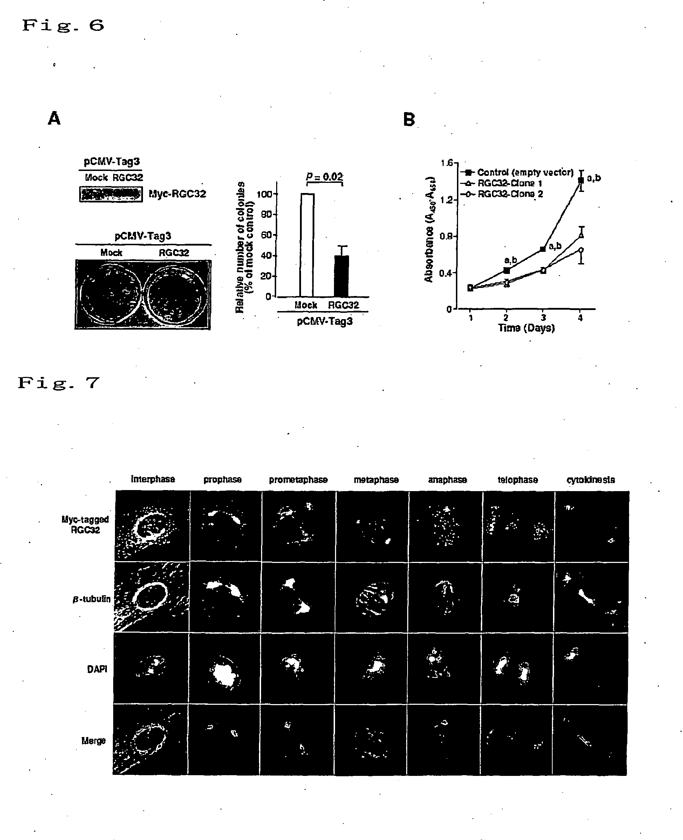

Fig. 6A (upper left) shows the results of transfecting U-87 MG cells (observed not to express the RGC32 gene) with a Myc-tagged RGC32 gene expression vector (pCMV-Tag3-RGC32) or a negative control (pCMV-Tag3-Mock) and then detecting the expression of the externally-transfected genes at 48 hours after transfection by the Western blotting method using 10 pig of each cell disruption solution and an anti-Myc antibody. Fig. 6A (lower left) shows photographs of culture plates obtained by culturing the 2 types of transfected cells (obtained under the above conditions) in medium in the presence of G418 for 3 weeks and then observing the colony formation ability via crystal violet staining. Fig. 6A (right) shows the summary of the results of observing colonies with a diameter of 2 mm or more to know the colony formation ability. Specifically, Fig. 6A (right) shows that the expression of the RGC32 gene caused a significant decrease in colony formation ability compared with the control. Fig. 6B shows the results of transfecting U-87 MG cells with the RGC32 gene (pCMV-Tag3-RGC32) and a negative control (pCMV-Tag3-Mock (empty vector)), establishing G418-resistant clones (2 types of RGC32 gene expression clone), and then detecting the cell proliferation rates (based on the number of viable cells) using chromaticity of a water-soluble tetrazolium salt.
Fig. 7 shows fluorescence microscopic images obtained by staining U-87 MG cells (observed not to express the RGC32 gene) transiently expressing a Myc-tagged RGC32 gene expression vector (pCMV-Tag3-RGC32) with an anti-Myc antibody (red), β-tubulin (green), or DAPI (blue), observing the cells throughout a period ranging from the interphase to the cytokinesis phase. Images in the lower case are merged fluorescence microscopic images thereof.

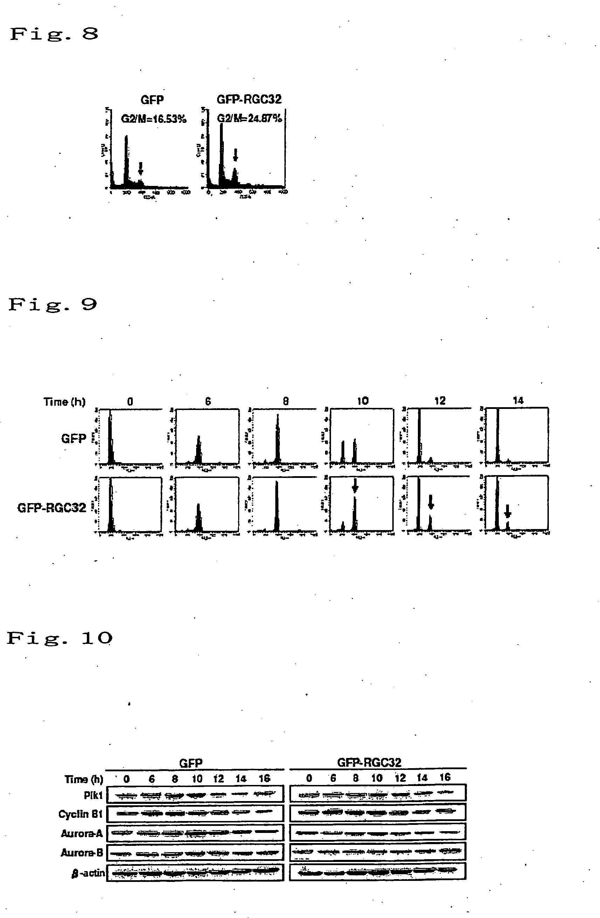

Fig. 8 shows the results of analyzing by FACS HeLa Tet-On cells after induction of GFP or GFP-RGC32 using Dox. Each arrow indicates 4N peak (G2/M phase).
Fig. 9 shows the results of analyzing by FACS HeLa Tet-On cells expressing GFP or GFP-RGC32 after the cancel of G1/S cell synchronization using double thymidine block. Each arrow indicates 4N peak (G2/M phase). Delayed transition from the M phase was observed in the HeLa Tet-On cells expressing GFP-RGC32 compared with the control cells expressing GFP.
Fig. 10 shows HeLa Tet-On cells expressing GFP or GFP-RGC32 after the cancel of G1/S cell synchronization using double thymidine block, as detected by the Western blotting method using antibodies against M-phase kinase Plk1, cyclin B1, Aurora-A, Aurora-B, and control β actin, respectively.

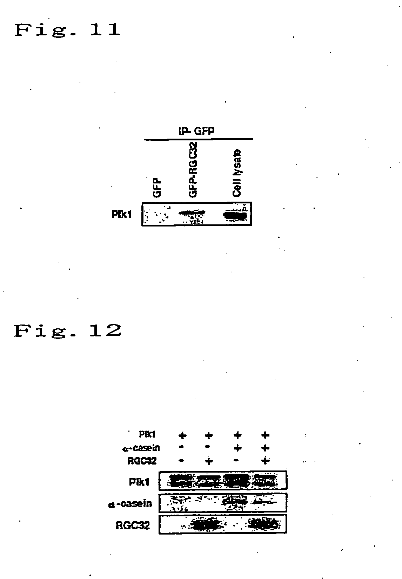

Fig. 11 shows the results of subjecting HeLa Tet-On cell disruption solutions (after induction of GFP or GFP-RGC32 with Dox) to immunoprecipitation using an anti-GFP antibody and then subjecting the resultants to detection by the Western blotting method using an anti-Plk1 antibody. "Cell lysate" indicates the result of detection using a control cell disruption solution.
Fig. 12 shows the results of preparing an RGC32 gene recombinant protein and then detecting Plk1 kinase activity with the use of a combination of human recombinant Plk1 and dephosphorylated α-casein by autoradiography.

### Preferred Embodiments of the Invention

The embodiments of and methods for implementing the present invention are described in detail as follows

### (1) Cancer suppressing agent of the present invention

According to an embodiment of the present invention, the agent for suppressing cancer of the present invention comprises an RGC32 gene or a gene homologous thereto as defined in the claims as an active ingredient. According to another embodiment of the present invention, the agent for suppressing cancer of the present invention comprises an RGC32 protein or a protein homologous thereto as an active ingredient.

The nucleotide sequence of the RGC32 gene and the amino acid sequence of the RGC32 protein are known (Badea, T. C., Niculescu, F. I., Soane, L., Shin, M. L., and Rus, H. 1998). The nucleotide sequence of the RGC32 gene has been registered as AF16963 in the database of the National Center for Biotechnology Information. The amino acid sequence of the RGC32 protein has been registered as NP054778 in the same database. The nucleotide sequence of the RGC32 gene is as shown in SEQ ID NO: 1. The RGC32 protein is encoded by a region between nucleotide 147 and nucleotide 500 of the nucleotide sequence of SEQ ID NO: 1. The amino acid sequence of the RGC32 protein is as shown in SEQ ID NO: 2

"RGC32 gene" in the Description refers to a human-derived gene that is specified by the above nucleotide sequence. "RGC32 protein" in the same refers to a protein that is encoded by the RGC32 gene and is specified by the above amino acid sequence.

The RGC32 gene may be cDNA that is obtained from cultured cells through the use of techniques known by persons skilled in the art or may be chemically synthesized by a PCR method or the like based on the nucleotide sequence of SEQ ID NO: 1 in the Description. When DNA having the nucleotide sequence shown in SEQ ID NO: 1 is obtained by the PCR method, PCR is performed using human chromosomal DNA or a cDNA library as a template and a primer set designed to enable amplification of the nucleotide sequence shown in SEQ ID NO: 1. The DNA fragment amplified by PCR can be cloned into an appropriate vector capable of amplification in a host such as *Escherichia coli.*

Procedures including preparation of the above probes or primers, cDNA library construction, cDNA library screening, cloning of a target gene, and the like are known by persons skilled in the art. For example, such procedures can be performed according to the methods described in Molecular Cloning: A Laboratory Manual, 2nd Ed., Cold Spring Harbor Laboratory, Cold Spring Harbor, NY, 1989; Current Protocols in Molecular Biology, Supplement 1 to 38, John Wiley & Sons (1987-1997) and the like.

In the present invention, "gene homologous to the RGC32 gene" refers to: a gene having a nucleotide sequence that is derived from the nucleotide sequence shown in SEQ ID NO: 1 by deletion, addition, or substitution of one to 60 nucleotides and encodes a protein having cancer-suppressing activity; or a gene having a nucleotide sequence that is capable of hybridizing to the nucleotide sequence shown in SEQ ID NO: 1 under stringent conditions and encodes a protein having cancer-suppressing activity.

A gene homologous to the RGC32 gene means a gene with between approximately 1 and 60, preferably a range between approximately 1 and 30, more preferably between approximately 1 and 20, further preferably a range between approximately 1 and 10, and particularly preferably a range between approximately 1 and 5 nucleotides deletions, additions, or substitutions of the nucleotide sequence shown in SEQ ID NO:1.

The degree of the term "cancer-suppressing activity" is not particularly limited, but refers preferably to cancer-suppressing activity that is substantially equivalent to or of a higher degree than the cancer-suppressing activity of the RGC32 protein. (Hereinafter, "cancer-suppressing activity" in the Description refers to the same.)

Therefore, a "gene homologous to the RGC32 gene" may be derived from any origin, as long as the homologous gene has a structure and functions as defined above. The homologous gene may be derived from a non-human mammal or a gene prepared by artificially introducing mutation into a gene derived from a mammal such as a human. However, when the homologous gene is used for a cancer suppressing agent as described later, such gene is preferably derived from a human in view of clinical safety.

The "gene that has a nucleotide sequence derived from the nucleotide sequence shown in SEQ ID NO: 1 by deletion, addition, or substitution of one to several nucleotides and encoding a protein having cancer-suppressing activity" can be prepared by any methods known by persons skilled in the art, such as chemical synthesis, gene engineering techniques, or mutagenesis. Specifically, the above gene can be obtained by introducing mutation into DNA using the DNA having the nucleotide sequence shown in SEQ ID NO: 1. The gene can be obtained through the use of a method that involves causing DNA having the nucleotide sequence shown in SEQ ID NO: 1 to come into contact with a drug that is a mutagen, a method that involves UV irradiation, and a gene engineering technique, for example. The site-directed mutagenesis which is one of the gene engineering techniques is a technique capable of introducing a specific mutation into a specific position. Thus, site-directed mutagenesis is useful and can be performed according to the method described in Molecular Cloning: A laboratory Manual, 2nd Ed., Cold Spring Harbor Laboratory, Cold Spring Harbor, NY, 1989; Current Protocols in Molecular Biology, Supplement 1 to 38, John Wiley & Sons (1987-1997) or the like.

The above "nucleotide sequence capable of hybridizing under stringent conditions" means a nucleotide sequence of DNA that is obtained by a colony hybridization method, a plaque hybridization method, or a Southern hybridization method using such DNA, as a probe. An example of such DNA can be identified by performing hybridization at 65°C in the presence of 0.7 M to 1.0 M NaCl using a filter on which colony- or plaque-derived DNA or a fragment thereof has been immobilized and then washing the filter under conditions of 65°C using a 0,1 to 2×SSC solution. (1×SSC solution comprises 150 mM sodium chloride and 15 mM sodium citrate.) Hybridization can be performed according to the method described in Molecular Cloning: A laboratory Manual, 2nd Ed., Cold Spring Harbor Laboratory, Cold Spring Harbor, NY, 1989 or the like.

An example of such DNA capable of hybridizing under stringent conditions is a DNA having a predetermined or higher degree of homology with the nucleotide sequence of DNA to be used as a probe. For example, such DNA has 70% or more, preferably 80% or more, more preferably 90% or more, further preferably 93% or more, and particularly preferably 95% or more homology.

The "gene that has a nucleotide sequence capable of hybridizing under stringent conditions to the nucleotide sequence shown in SEQ ID NO: 1 and encoding a protein having cancer-suppressing activity" can be obtained by performing a colony hybridization method, a plaque hybridization method, or a Southern hybridization method under certain hybridization conditions as described above

In the present invention, the term "protein homologous to the RGC32 protein" refers to: a protein having an amino acid sequence that is derived from the amino acid sequence shown in SEQ ID NO: 2 by deletion, substitution, and/or insertion of one to several amino acids and has cancer-suppressing activity; or a protein having an amino acid sequence that has 70% or more homology with the amino acid sequence shown in SEQ ID NO: 2 and has cancer-suppressing activity.

The range of the term "one to several" in the above "an amino acid sequence derived from the amino acid sequence shown in SEQ ID NO: 2 by deletion, substitution, and/or insertion of one to several amino acids" is not particularly limited. For example, the range of the term "one to several (amino acids)" refers to a range between approximately 1 and 20, preferably a range between approximately 1 and 10, more preferably a range between approximately 1 and 7, further preferably a range between approximately 1 and 5, and particularly preferably a range between approximately 1 and 3 amino acids.

The above "amino acid sequence having 70% or more homology with the amino acid sequence shown in SEQ ID NO: 2" means that the homology between the amino acid sequence and the amino acid sequence shown in SEQ ID NO: 2 is at least 70%, preferably 80% or more, and more preferably 90% or more.

The RGC32 protein may be a protein derived from the nature, a chemically synthesized protein, or a recombinant protein prepared by gene recombination technology. A recombinant protein is preferable in that it can be produced in large amounts by relatively easy procedures.

Such a protein derived from the nature can be isolated through the use of an adequate combination of protein isolation methods and protein purification methods from cells or tissues expressing the protein. Such a chemically synthesized protein can be synthesized according to a chemical synthesis method such as Fmoc method (fluorenylmethyloxycarbonyl method) or tBoc method (t-butyloxycarbonyl method). Moreover, the protein of the present invention can also be synthesized using various commercial peptide synthesizers. A recombinant protein can be produced by introducing DNA having a nucleotide sequence (e.g., the nucleotide sequence shown in SEQ ID NO: 1) encoding the protein into a suitable expression system.

In addition, a protein having an amino acid sequence derived from the amino acid sequence shown in SEQ ID NO: 2 by deletion, substitution, or insertion of one to several amino acids or a protein having an amino acid sequence that has 70% or more homology with the amino acid sequence shown in SEQ ID NO: 2 can be adequately produced or obtained by persons skilled in the art based on information concerning the nucleotide sequence shown in SEQ ID NO: 1, which is an example of a DNA, sequence encoding the amino acid sequence shown in SEQ ID NO: 2.

In a preferred embodiment of the cancer suppressing agent of the present invention, the cancer suppressing agent contains as an active ingredient a recombinant vector into which the above RGC32 gene or a gene homologous thereto has been incorporated. As a vector, a viral vector or a vector for expression in animal cells is used. Preferably, a viral vector is used.

Examples of a viral vector include a retrovirus vector, an adenovirus vector, an adeno-associated virus vector, a baculovirus vector, a vaccinia virus vector, and a lentivirus vector. Particularly, the use of a retrovirus vector is desired because the viral genome is incorporated into the host chromosome after infection of cells with the retrovirus vector so as to enable stable and long-term expression of the foreign gene incorporated in the vector.

Examples of a vector that can be used for expression in animal cells include pCXN2 (Gene, 108, 193-200, 1991), PAGE207 (JP Patent Publication (Kokai) No. 6-46841 A (1994)), and altered products thereof.

The above recombinant vector can be produced by introducing the vector into an appropriate host for transformation and then culturing the thus obtained transformant. When a recombinant vector is a viral vector, animal cells capable of producing viruses are used as hosts into which such vector is introduced. Examples of such animal cells include COS-7 cells, CHO cells, BALB/3T3 cells, and HeLa cells. Examples of hosts to be used for retroviral vectors include ΨCRE, ΨCRIP, and MLV. Examples of hosts to be used for adenovirus vectors or adeno-associated virus vectors include 293 cells derived from human fetal kidney. Viral vectors can be transfected into animal cells via a calcium phosphate method or the like. Furthermore, when a recombinant vector is a vector for expression in animal cells, examples of hosts that can be used for introduction of such vector include the *Escherichia coli* K12 strain, the HB 101 strain, and the DH 5α strain. Transformation of *Escherichia coli* is known by persons skilled in the art. The thus obtained transformants are each cultured in an adequate medium under adequate culture conditions. For example, a transformant of *Escherichia coli* can be cultured using a liquid medium with a pH approximately between 5 and 8 containing carbon sources, nitrogen sources, inorganic matter, and the like, which are necessary for growth. In general, culture is carried out at 15°C to 43°C for approximately 8 to 24 hours. In such case, a recombinant vector of interest can be obtained after the termination of culture via general DNA isolation and purification methods.

Furthermore, transformants of animal cells can be cultured using a medium such as a 199 medium, an MEM medium, or a DMEM medium containing approximately 5% to 20% fetal calf serum, for example. The pH of such medium is preferably approximately between 6 and 8. In general, culture is carried out at approximately 30°C to 40°C for approximately 18 to 60 hours. In such case, since viral particles containing a recombinant vector of interest are released into a culture supernatant, the recombinant vector of interest can be obtained as a result of concentration and purification of viral particles via a cesium chloride centrifugation method, a polyethylene glycol precipitation method, a filter concentration method, or the like

Of the agents for suppressing cancer of the present invention, the agent (hereinafter, referred to as gene therapeutic agent) containing as an active ingredient the RGC32 gene or a gene homologous thereto can be produced by mixing the RGC32 gene or the gene homologous thereto as an active ingredient with a base that is generally used for a gene therapeutic agents. Moreover, when the RGC32 gene or the gene homologous thereto is incorporated into a viral vector, viral particles containing a recombinant vector are prepared and the viral particles are mixed with a base that is generally used for gene therapeutic agents.

As the above base, a base generally used for an injection can be used. Examples thereof include: distilled water; a salt solution of sodium chloride, a mixture of sodium chloride and an inorganic salt, or the like; a solution of mannitol, lactose, dextran, glucose, or the like; an amino acid solution of glycine, arginine, or the like; and a mixed solution of an organic acid or salt solution and a glucose solution. Alternatively, in accordance with conventional techniques known by persons skilled in the art, an injection can be prepared as a solution, suspension, or dispersion using an adjuvant such as an osmoregulator, a pH adjuster, a plant oil, or a surfactant with the above base. Such injection can also be prepared as a pharmaceutical preparation that is solubilized at the time of use through operations such as pulverization and freeze drying.

Furthermore, the gene therapeutic agent of the present invention can also be produced by adding the RGC32 gene to a liposome suspension prepared according to a standard method, freezing the mixture, and then thawing the frozen mixture. Examples of a method for preparing liposomes include a thin-film agitation method, an ultrasound method, a reverse-phase evaporation method, and a surfactant removal method.

Preferably, a liposome suspension is subjected to ultrasonication and then the gene is added so as to improve the encapsulation efficiency of the gene. Liposomes in which the gene has been encapsulated can be directly administered intravenously or can be suspended in water, a physiological saline solution, or the like and then administered intravenously.

The route of administration of the gene therapeutic agent may involve a form of general systemic administration such as intravenous administration or intraarterial administration. Alternatively, the route of administration may also involve a form of local administration such as local injection or oral administration to oncogenic lesions or presumed metastatic sites. Furthermore, the gene therapeutic agent can also be administered through a combination of catheterization, a transfection technique, surgical operation, and the like.

The dose of the gene therapeutic agent varies depending on patient age, sex, and symptoms, the route of administration, the number of doses, and dosage forms. In general, the daily dose (in terms of recombinant gene weight) ranges from 1 µg/kg body weight to 1000 mg/kg body weight for an adult. Preferably, it ranges from 10 µg/kg body weight to 100 mg/kg body weight. The number of doses is not particularly limited.

Of the agents for suppressing cancer of the present invention, the agent (hereinafter, referred to as a protein drug) containing as an active ingredient the RGC32 protein or a protein homologous thereto as defined in the claims can be provided in the form of a pharmaceutical composition containing the RGC32 protein or the protein homologous thereto as an active ingredient and additives for pharmaceutical preparations (e.g., a carrier and an excipient).

The form of the protein drug is not particularly limited. Examples of such form for oral administration include tablets, capsules, granules, powders, fine granules, liquids, and syrups. Examples of such form for parenteral administration include injections, infusions, suppositories, inhalants, transmucosal absorption systems, and transdermal absorption systems.

The route of administration for the protein drug is not particularly limited and may involve a form of oral administration or parenteral administration (e.g., intramuscular administration, intravenous administration, subcutaneous administration, peritoneal administration, transmucosal administration, and inhalation administration).

The dose of the protein therapeutic agent varies depending on patient age, sex, and symptoms, the route of administration, the number of doses, and dosage forms. In general, the daily dose ranges from 0.001 µg/kg body weight to 1000 µg/kg body weight for an adult. Preferably, it ranges from 0.001 µg/kg body weight to 100 µg/kg body weight. The number of doses administered is not particularly limited.

The above-mentioned cancer suppressing agents (including both gene therapeutic agent and protein drug forms thereof) can be used for suppressing cancer through administration of the effective dose thereof to mammals including humans. The above cancer suppressing agents can also be used for preventing and/or treating cancer through administration of prophylactically and/or therapeutically effective doses thereof to mammals including humans.

"Cancer suppression (cancer suppressing)" in the Description is interpreted in the broadest sense such that it refers to both a prophylactic effect of preventing the onset of cancer, cancer metastasis, and/or cancer implantation and a therapeutic effect of blocking the advancement of cancer through suppression of cancer cell proliferation or diminishing of cancer, so as to alleviate the symptoms. Hence, "cancer suppression (cancer suppressing)" is never interpreted in a limited way in any case.

Specific examples of cancer to which the cancer suppressing agents of the present invention are applied include, but are not limited to, malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract cancer, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma. In the present invention, a particularly preferable type of cancer to which the cancer suppressing agents are applied is brain tumor.

### (2) Method for diagnosing cancer using the RGC32 gene

Examples of the method for diagnosing cancer include a method for diagnosing cancer malignancy and a diagnostic method for selecting cancer to which the agents of the present invention are applied. The method for diagnosing cancer comprises the step of analyzing the RGC32 gene in a specimen sample using DNA or RNA containing the whole or a portion of the RGC32 gene.

"A portion of the RGC32 gene" used herein means an oligonucleotide comprising a nucleotide sequence of approximately 10 to 30 sequential nucleotides, for example, in the nucleotide sequence of the RGC32 gene shown in SEQ ID NO: 1 Examples of specimen samples that can be used herein include tissue sections, blood, lymph fluids, sputum, lung lavage fluids, urine, stool, and tissue culture supernatants, which are suspected of being affected by tumor.

The above "detection for selecting cancer to which the cancer suppressing agents are applied" refers to determination of the presence or the absence of cancer in tissue or the like against which the cancer suppressing agents of the present invention can effectively act.

Cancer is diagnosed by analyzing the RGC32 gene in a specimen sample using DNA or RNA containing the whole or a portion of the RGC32 gene as a primer or a probe. "Analyzing the RGC32 gene" used herein specifically refers to detection of deletion of the relevant gene from genomic DNA or detection of an abnormality in the expression level of the gene.

When the above DNA or RNA is used as a primer, gene mutation can be detected by amplifying a partial DNA sequence prepared from a specimen sample by the PCR method using two selected primer sequence types and then confirming the presence or the absence thereof, for example. Alternatively, gene mutation can also be detected by directly confirming the amplified product or confirming the sequence thereof after its recombination into various plasmid vectors.

Meanwhile, an abnormality in gene expression level can be detected by a Northern hybridization method or an RT-PCR (reverse transcription-polymerase chain reaction) method using a probe containing the above RNA sequence.

### (3) Method for diagnosing cancer using the RGC32 protein antibody or a fragment thereof

The method for diagnosing cancer comprises the step of analyzing the amount of the RGC32 protein in a specimen sample using an antibody against the RGC32 protein or a fragment thereof.

Such antibody (hereinafter, referred to as an RGC32 antibody) against the RGC32 protein, which is used in the aforementioned method, can be prepared by a general method using the whole or a portion of the RGC32 protein as an antigen. "A portion of the RGC32 protein" used herein refers to a polypeptide comprising at least 6 sequential amino acids, preferably at least approximately 8 to 10 amino acids, and further preferably at least approximately 11 to 20 amino acids in the amino acid sequence of the RGC32 protein shown in SEQ ID NO: 2. A method for preparing the whole or a portion of the RGC32 protein, which is used as an antigen, may be either a biological technique or a chemical synthesis technique.

For example, a polyclonal antibody can be prepared by injecting the above antigen in several instances into animals such as mice, guinea pigs, or rabbits subcutaneously, intramuscularly, intraperitoneally, intravenously, or the like, sufficiently immunizing the animals, collecting blood from the animals, and then separating out serum. For example, a monoclonal antibody can be prepared by preparing a hybridoma that is obtained via cell fusion of a spleen cell of a mouse into which the above antigen has been injected with a commercial mouse myeloma cell and then preparing the monoclonal antibody from the culture supernatant of the hybridoma or the ascite of a mouse to which the hybridoma has been administered.

The expression level of the RGC32 protein in a specimen sample can be measured using the RGC32 protein antibody or a fragment thereof prepared as described above. For measurement, an immunological technique such as an immunoblot technique, an enzyme immunoassay (EIA) method, a radio immunoassay (RIA) method, a fluorescent antibody technique, immunocytological staining, or a Western blot method can be used, for example. "A fragment of the RGC32 protein antibody" used herein refers to a single-chain antibody fragment (scFv) of the antibody, for example. Moreover, examples of specimen samples that can be used herein include tissue sections, blood, lymph fluids, sputum, lung lavage fluids, urine, stool, and tissue culture supernatants, which are suspected of being affected with tumor. When a low expression level of the RGC32 protein in a specimen sample is detected, this indicates suppresses expression of the RGC32 gene in a tissue or a cell specimen. Hence, cancers to which the agents of the present invention are applied can be selected.

Hereafter, the present invention is described in greater detail with reference to the following examples, although the present invention is not limited to these examples.

### Examples

### (1) Experimental materials

Glioma-derived cell lines (A-172, AM-38, Becker, GB-1, KALS-1, KINGS-1, KNS-42, KNS-60, KNS-81,KNS-89, KS-1, Marcus, NMC-G1, no. 10, no. 11, SF126, T98G, U-251 MG, YH-13, and YKG-1) were obtained from the National Institute of Biomedical Innovation (JCRB) and then used. U-373 MG, U-87 MG, and SaOS2 were obtained from the American Type Culture Collection (ATCC). HeLa Tet-On cells of cervical cancer were obtained from Clontech. Large bowel cancer cell lines (HCT116 (p53+/+) and HCT116 (p53-/-)) were provided by Dr. Bert Vogelstein as a favor.
These cell lines were cultured in Dulbecco's modified Eagle's medium (DMEM) in the presence of 10% fetal calf serum, 100 units/ml penicillin, and 100 µg/ml streptomycin. Astroglioma samples of 35 patients (from which clinical specimens were obtained) were obtained from the Department of Neurosurgery at the Tokyo Medical and Dental University Hospital Faculty of Medicine and then used under agreement with each patient and with the approval of the ethical committees of the relevant organizations. Staging with the use of hernatoxylin-eosin staining was performed according to World Health Organization (WHO) standards. Of 35 carcinoma samples, 8 samples were determined to be low-grade diffuse astrocytoma (Grade II), 8 samples were determined to be anaplastic astrocytoma (Grade III), and 19 further samples were determined to be glioblastoma multiforme (GBM, Grade IV). Table 1 is a list of the tissue information on the glioma-derived cell lines.

**Table 1:**

| 22 glioblastoma-derived cell lines | | |
|---|---|---|
| **Cell line** | **Histology*** | **Description** |
| 1 A-172 | Glioblastoma | |
| 2 AM-38 | Glioblastoma | GFP and S-100-positive, ACNU (a cancer chemotherapy drug)-sensitive |
| 3 Becker | Astrocytoma | GFAP-negative |
| 4 GB-1 | Glioma | MDR1 and P-glycoprotein-expressing, |
| | | GFAP, vimentin and fibronectin-positive |
| 5 KALS-1 | Glioma | GFAP, vimentin and CD 13-positive |
| 6 KINGS-1 | Anaplastic | GFAP, S-100, vimentin, CD 13 and |
| | astrocytoma | HNK-1-positive |
| 7 KNS-42 | Glioma | GFAP-positive, S-100 and NSE-negative |
| 8 KNS-60 | Glioma | GFAP, S-100 and NSE-negative |
| 9 KNS-81 | Glioma | GFAP and S-100-positive, NSE-negative |
| 10 KNS-89 | Gliosarcoma | GFAP and NSE-positive, S-100-negative |
| 11 KS-1 | Gliosarcoma | |
| 12 Marcus | Astrocytoma | GFAP-negative |
| 13 NMC-G1 | Glioma | FGF-9-producing |
| 14 No. 10 | Anaplastic glioma | GFAP-positive |
| 15 No. 11 | Anaplastic glioma | GFAP-positive |
| 16 SF126 | Glioblastoma | GFAP-negative |
| 17 T98G | Glioblastoma | Anchorage-independent |
| 18 U-251 MG | Astrocytoma | GFAP-positive |
| 19 U-373 MG | Glioblastoma- | |
| | astrocytoma | |
| 20. U-87 MG | Glioblastoma- | |
| | astrocytoma | |
| 21 YH-13 | Gliosarcoma | GFAP and S-100-positive, ACNU (a cancer chemotherapy drug)-resistant |
| 22 YKG-1 | Gliosarcoma | GFAP and S-100 protein identified. HSR on marker chromosome identified. α and β-tumor growth factors expressed |

| | | |
|---|---|---|
| * Histological subtype of the primary tumor from which each cell was derived. | | |

### (2) Separation of low-expression gene in glioma-derived cells by a microarray method for expression analysis

The results of screening for DNA-amplified genes and DNA-deleted genes of the above 22 types of glioma-derived cells via an array CGH method using MCG Cancer Array-800 have been reported (Saigusa K., et al., Cancer Sci. 96, 676, 2005). At this time, high-frequency hemizygous deletion was observed in the long arm of chromosome 13. Accordingly, messenger RNA expression analysis was carried out using RNA derived from normal human brain, and RNA derived from Marcus cells, and AceGene Human oligo chip 30K (DNA Chip Research Inc.) capable of observing expression levels of 30,000 human genes. Hence, low-expression genes in the long arm of chromosome 13 were searched. A complementary strand DNA (complementary DNA)
in which aminoallyl dUTP (Ambion Inc.) had been incorporated was prepared with the use of oligo(dT) 12-18 primer. A coupling reaction was conducted with Cy3 (cyanine 3, Marcus) and Cy5 (cyanine 5, normal human brain) (Amersham Biosciences) that were reactive to an aminoallyl group so that labeling was performed. After hybridization, signals were measured using GenePix 4000B (Axon Instruments) and then the results were analyzed by GenePix Pro 6.0 software (Axon Instruments). Table 2 shows genes in the long arm of chromosome 13, the expression levels of which were lowered in Marcus cells as compared with those in cells derived from the normal human brain.

**Genes the expression of which were lowered because of the deletion of chromosome 13q**

| | | | | | |
|---|---|---|---|---|---|
| | | | | | |

| **Acc. Numbed** | **LocusLink ID** | **Location** | **Symbol** | **Gene Name** | **log2ratio*)** |
|---|---|---|---|---|---|
| NM_024026_1 | 78988 | 13p11.1-q11 | MRP63 | mitochondrial ribosomal protein 63 | -0,99 |
| NM_001629_1 | 241 | 13q12 | ALOX5AP | arachidonate 5-lipoxygenase-activating | -1,61 |
| NM_014059_1 | 28984 | 13q14.11 | RGC32 - | RGC32 protein | -0,83 |
| NM_000504_1 | 2159 | 13q34 | F10 | coagulation factor X | -1,22 |

| | | | | | |
|---|---|---|---|---|---|
| *) Fluorescence intensity of Cy-3-labeled Marcus / fluorescence intensity of Cy-5-labeled normal brain sample | | | | | |

Messenger RNA expression of RGC32-containing gene in the long arm of chromosome 13

To measure the messenger RNA expression levels of the RGC32 gene and MRP63, ALOX5AP, RGC32, and F10 genes, for which lowered expression levels had been observed in the long arm of chromosome 13 by an expression analysis microarray, RT-PCR was performed for 22 glioma-derived cell lines using the normal brain as a control (Fig. 1). GAPDH was used as a control showing a control expression level in RT-PCR, since the expression level thereof is known to hardly fluctuate depending on cell types or conditions. Expression analysis was conducted for the RGC32 gene and

MRP63, ALOX5AP, and F10 genes. In particular, significantly lowered expression levels of the RGC32 gene were observed in 21 out of 22 cell lines.

35 astrocytoma samples were pathologically classified. Quantitative RT-PCR was performed for the RGC32 gene using GAPDH as a control. 8 samples of low-grade diffuse astrocytoma (Grade II), 8 samples of anaplastic astrocytoma (Grade III), and 19 samples of glioblastoma multiforme (GBM, Grade IV) were separately subjected to Student's t-test. As a result, a significant inverse correlation was observed between grades representing pathological malignancies (Grade II to Grade IV) and RGC32 gene expression levels (Fig. 2A, p = 0.003). Moreover, the relationship between the presence or the absence of p53 gene point mutation (p53 gene is a typical cancer suppressing gene) and RGC32 gene expression levels was examined for each grade determined based on the pathological classification (Fig. 2B). As a result, p53 gene point mutation was observed to be present regardless of grades. Furthermore, RGC32 gene expression levels were observed to have a tendency to decrease in those with p53 gene point mutation, although there were no significant differences.

### (3) RGC32 gene expression in cells forced to express p53 gene

A U-373 MG cell line (in which p53 gene is unable to function as is known) was infected with adenovirus expressing a human p53 gene. RGC32 gene expression was observed. Human β-galactosidase (Ad-lacZ) was used as a control. Both viruses (p53 (Ad-p53) and Ad-lacZ) were separately used for infection of cells, and then expression level of RGC32 gene messenger RNA were measured by RT-PCR (Fig. 3A). GAPDH was used as a control. A p21 gene is known such that the expression level thereof is elevated because of the expression of the p53 gene. It was also confirmed that the expression levels of the RGC32 gene were elevated after infection with the adenovirus expressing the p53 gene, in a manner similar to that in the case of the p21 gene. On the other hand, no elevated expression levels of both p21 and p53 genes were confirmed in cells infected with human β-galactosidase (Ad-lacZ).

### (4) RGC32 gene expression and RGC32 protein expression in DNA cells

Adriamycin (ADR) (an anticancer agent inducing DNA damage) was added at a concentration of 1µg/ml to both the large bowel cancer cell lines: HCT116 (p53+/+) having the endogenous p53 gene; and HCT116 (p53-/-) lacking the endogenous p53 gene. The expression levels of the p21 and p53 proteins were measured by a Western blotting method (upper) and the messenger RNA expression of the p21 and RGC32 genes were measured by an RT-PCR method (Fig. 3B). It is broadly known that the provision of DNA damage to cells results in elevated expression levels of both the p53 and p21 genes. Induction of the normal p53 protein was observed only in the HCT116 cells (p53+/+) having the normal p53 protein. Regarding messenger RNA expression of the p21 and RGC32 genes, elevated expression levels of the messenger RNA were observed only in the HCT116 cells (p53+/+) having the normal p53 protein after addition of ADR. It was confirmed from these results that the expression level of the RGC32 gene was elevated via the normal p53 protein.

### (5) Binding site of p53 protein on RGC32 gene DNA

The nucleotide sequence of the RGC32 gene on the genomic DNA was confirmed. The presence of the binding sites of the p53 protein comprising approximately 20 nucleotide pairs was examined. 10 binding sites of the p53 protein with 80% or more homology (el-Deiry WS, et al., Nat Genet. 1. 45. 1992) were confirmed (Fig. 3C). The binding sites were designated RGC32-RE1 to RGC32-RE10.

### (6) Transcription-promoting effect of a promoter region in the RGC32 gene as measured by reporter assay

Promoter activity was confirmed for each of 10 binding sites of the p53 protein of the RGC32 gene on the genomic DNA, RGC32-RE1 to RGC32-RE10 by a method for measuring light-emitting substances using a firefly luciferase enzyme. Constructs having a luciferase gene located in a downstream region using a pGL3 vector (Promega) and containing promoter analysis regions (RGC32-RE1 to RGC32-RE10) were prepared. A vector (pCMV-Tag3-p53) expressing a wild-type p53 protein, a vector expressing a p53 mutant protein as a control, and a vector lacking the p53 gene (pCMV-Tag3-p53Mut and pCMV-Tag3-mock) were incorporated in combination into SaOS2 cells lacking the p53 gene. Enzyme activity was then determined. As a result, only in the case of RGC32-RE2, luciferase enzyme activity levels were elevated only when the wild-type p53 protein was expressed (data not shown). Hence, RGC32-RE2 was synthesized by ligation of a synthetic oligonucleotide (5'-AGGCgAGTTT-aag-cAGCTTGTCC-3') once or repeatedly (twice) and then inserted as a pGL3 vector promoter. The resultant was transfected into SaOS2 cells with pCMV-Tag3-p53, pCMV-Tag3-p53Mut (R273H), or pCMV-Tag3-mock. 36 hours later, luciferase enzyme activity was determined (Fig. 4). In the case of p53 Consensus Binding Site (CBS) derived from the p21 gene, luciferase enzyme activity levels were elevated in the presence of a vector expressing the wild-type p53 protein. Similar to such results, a luciferase enzyme activity level 60 times higher than that in the case of the control was confirmed in the case of RGC32-RE2 and particularly RGC32-RE2x2 (synthesized via 2 times of ligation). It was confirmed that this sequence functions as a promoter region in a manner depending on the p53 protein.

### (7) Confirmation of the presence of promoter DNA by Chromatin immunoprecipitation (ChIP)

ChIP assay was performed using a ChIP Assay Kit (Upstate Biotechnology). 2 × 10⁶ HCT116 (p53+/+) cells and HCT116 (p53-/-) cells were seeded on a 10 cm dish. ADR was added at a final concentration of 1 µg/ml. 24 hours later, cells were treated with 1% formamide for 10 minutes. The resulting cells were harvested in 200 µl of SDS cell lysis buffer containing a protease inhibitor cocktail (Roche). Ultrasonication was performed so that the cells were disrupted to result in DNA lengths ranging from 200 bp to 1000 bp. The supernatants were treated with salmon sperm DNA/protein agarose. Immunoprecipitation was performed at 4°C for 16 hours using an anti-p53 antibody (Ab-6; Oncogene Research Products) or an anti-FLAG antibody (M2; Sigma) as a control. Each precipitate was dissolved in 50 µl of Tris-EDTA. Each p53 binding site was detected by PCR using 1 µl of each of the thus obtained solution as a template. The presence of the promoter sequence was observed only in the HCT116 (p53+/+) that had been subjected to ADR treatment and immunoprecipitation using the anti-p53 antibody (Fig. 5, p21, RGC32-RE2). In addition to the result, upon actual intracellular DNA damage response, the response to the promoter sequence mediated by the p53 protein was also observed on RGC32-RE2.

### (8) Lowered carcinogenicity levels due to transfection of RGC32 gene into glioma cells

The effect of the transfection of the RGC32 gene into glioma cells on the cell proliferation activity was examined by confirming the presence or the absence of any changes in colony formation ability under anchorage-dependent conditions. First, a vector was constructed by inserting RGC32 full-length cDNA into a pCMV vector (Stratagene) in which a myc peptide had been added to the amino N-terminus of an insertion gene. Furthermore, a control vector lacking such insertion gene was also constructed. Specifically, pCMV-Tag3-RGC32 and pCMV-Tag3-Mock were constructed. These vectors were transfected into U-87 MG cells which show no expression of the RGC32 gene. At 48 hours after transfection, myc protein expression was observed using the cell lysis solutions by the Western blotting method. The presence of the pRGC32-Myc fusion protein was confirmed only in the cells transfected with pCMV-Tag3-RGC32, as predicted (Fig. 6, A, upper). Cells that had been cultured for 3 weeks in the presence of G418 were separately immobilized on a dish and then stained with crystal violet. Colonies were then measured. Unlike Mock, it was confirmed that the expression of the RGC32 gene causes significantly lowered colony counts (Fig. 6, A, lower).

### (9) Lowered proliferation ability levels due to transfection of the RGC32 gene into glioma cells

The effect of the transfection of the RGC32 gene into cell on the cell proliferation activity was examined by confirming the presence or the absence of changes in proliferation rate. pCMY-Tag3-RGC32 and pCMV-Tag3-Mock were transfected into U-87 MG cells whih show no expression of the RGC32 gene. Cells were cultured in the presence of G418 for 3 weeks so that cells constitutively expressing myc-RGC32 and negative control cells were established. The proliferation rates of these cells were examined as follows. The number of these cells were monitored (Dojindo Laboratories: cell counting kit-8) (Fig. 6B) through conversion to a formazan dye by mitochondria dehydrogenase in viable cells using a tetrazolium salt (WST-1). 1 × 10³ cells were seeded on a 96-well plate and then cultured. Unlike control cells (empty), cell proliferation rates were clearly decreased in both clone 1 and clone 2 because of the expression of the RGC32 protein after 3 days of culture. It was revealed by these results that the RGC32 protein is capable of suppressing cell proliferation through its expression within cells. Known cancer suppressing gene proteins protect the living body from cancer by suppressing cell proliferation or inducing cell death. Hence, it was demonstrated that one of the functions of the RGC32 protein is to function as a cancer suppressing gene product.

### (10) Analysis of RGC3 2 protein accumulation sites in cells undergoing transient expression

To cause the expression of the RGC32 protein having a Myc antigen, pCMV-Tag3-RGC32 was transfected into 10⁴ U-87 MG cells on a 8-well slide glass. 24 hours later, fixation was performed using cold acetone methanol (1:1). 2 hours of reaction was performed using a blocking solution (3% BSA-containing phosphate-buffered saline, PBS), an anti-Myc antibody (1 : 200), and an anti-β-tubulin antibody (1 : 200, Sigma). Subsequently, FITC-labeled sheep-derived anti-mouse IgG (1 : 400, MBL) and Alexa 594-labeled sheep-derived anti-rabbit IgG (1 : 1000, Molecular Probes) were caused to react for 1 hour. Contrast staining for staining cell nuclei was performed using 4', 6-diamidino-2-phenylindole (DAPI) and then the results were observed using an ECLIPSE E800 fluorescence microscope (Nikon Corporation) (Fig. 7). In terms of cell cycles, the RGC32 protein that had been transiently expressed during the interphase excluding the mitotic phase (M phase) was widely distributed on the cytoplasm. In particular, distribution of the RGC32 protein in the nuclear envelope was significantly observed. In the M phase, distribution of the RGC32 protein in the centrosome was observed during the prophase, the most intense staining result was observed throughout a period ranging from the prometaphase to the metaphase. In the case of cells in the telophase and cytokinesis, the RGC32 protein was observed at low levels in the centrosome. Thus, it was revealed that the RGC32 protein functions mainly in the centrosome.

### (11) Observation of the effect of the RGC32 protein on cell cycle

An expression vector (pTRE2-GFP-RGC32) in which RGC32 cDNA had been inserted together with a Green fluorescence protein (GFP) gene into pTRE2hyg was constructed. Thus HeLa Tet-On cells (Clontech) were established. At the same time, control cells were also established using a vector (pTRE2-GFP) containing GFP alone. After addition of doxycycline (Dox), the presence of the GFP protein was confirmed by the Western blotting method using a cell lysis solution and an anti-GFP antibody (MBL). 10⁴ cells were seeded on a 10-cm culture plate. 24 hours later, Dox (10 µg/ml) was added and then the cells were further cultured for 24 hours. The cells were fixed with 70% cold ethanol and then subjected to RNase A treatment and propidium iodide staining. Fluorescence was measured by fluorescence activated cell sorting (FACS; FACSCalibur HG (Becton-Dickinson)), and analysis was carried out using BD CellQuest^{™} Pro (Becton-Dickinson) (Fig. 8). As a result, it was revealed that the content of G2/M-phase-cells was higher (24.87%) in cells expressing GFP-RGC32 than that (16.53%) in the control cells. These results demonstrated that the RGC32 protein has an effect of increasing the number of G2/M-phase cells in terms of cell cycle.

### (12) Effect of RGC32 protein expression to extend the G2/M phase

2 types of HeLa Tet-On cell (GFP-RGC32 and GFP) in the logarithmic growth phase were seeded at 10⁶ cells per 10-cm culture plate. 24 hours later, cells were cultured in a culture solution containing thymidine (Sigma) with a final concentration of 10 mM and 5% Fetal Bovine Serum (FBS) at 37°C for 15 hours. After 3 times of washing with PBS, the cells were cultured for 9 hours in general culture solutions and then cultured again in the presence of thymidine with a final concentration of 10 mM for 15 hours. After 3 times of washing with PBS, the cells were treated in the presence of Dox (10 µg/ml) for different lengths of time until 24 hours of culture in general culture solutions. The resulting cells were analyzed by FACS (Fig. 9). As a result, in the case of GFP-RGC32 compared with that of GFP, the cell content in the G2/M phase was higher at (and after) 10 hours after Dox addition. It was demonstrated that the extension of the G2/M phase had taken place because of RGC32 protein expression.

### (13) Verification of interaction between RGC32 protein and Plk1 kinase

G2/M-phase to G1-phase transition was delayed by RGC32 protein expression and distribution of the RGC32 protein was observed mainly in the centrosome during the M phase. Hence, whether or not the RGC32 protein bound to a mitotic protein kinase was examined. HeLa Tet-On cells (GFP-RGC32, GFP) were treated with Dox for different lengths of time. The resulting cell lysis solutions of HeLa Tet-On cells (GFP-RGC32, GFP) were each lysed in a NP-40 buffer (50 mM Tris-HCl, pH 7.5, 150 mM NaCl, 1 mM EDTA, 0.5% Nonidet P-40, 2 mM Na3VO4, 100 mM NaF, and 10 mM sodiumdiphosphate decahydrate) in the presence of a protease-inhibitor cocktail. The resultants were analyzed by a standard method that is the Western blotting method using an anti-polo-like kinase 1 (Plk1) antibody (Zymed Laboratories), an anti-cyclin B1 antibody (Upstate), an anti-Aurora-B kinase antibody (Abcam), an anti-Aurora-A kinase antibody (provided by Mr. Hideyuki Satani), and an anti-β actin antibody as a control (Fig. 10). As a result, it was demonstrated that Plk1 degradation alone was delayed and specifically, Plk1 degradation was delayed by the RGC32 protein so as to cause the extension of the G2/M phase. Moreover, HeLa Tet-On cells (GFP-RGC32, GFP) at 24 hours after addition of Dox were lysed in NP-40 buffer. The cell lysis solutions were treated with an anti-GFP antibody at 4°C for 2 hours and then treated with Protein A Sepharose at 4°C for 1 hour. The resultants were washed 5 times with NP-40 buffer and then analyzed by the Western blotting method using an anti-Plk1 antibody (Fig. 11). As a result, binding of Plk1 due to the RGC32 protein that had bound to GFP which was immunoprecipitated with the GFP RGC32 only Furthermore, the effect of the RGC32 protein on Plk1 protein kinase activity was examined. Full-length RGC32 cDNA was inserted into pET-23d (Novagen) (pET-23d-RGC32). A recombinant His6-tagged RGC32 protein was produced in BL21-CodonPlus (DE3) (Stratagene) *Escherichia coli,* purified using a NI-NTA superflow affinity resin (Qiagen), and then dialyzed with PBS. Human recombinant Plk1 (Invitrogen) and recombinant His6-tagged RGC32 protein were incubated in the presence of dephosphorylated α-casein (Sigma) at 4°C for 30 minutes in Raf buffer (20 mM Tris-HCI (pH7.4), 10 mM MgCl₂, 0.1 mM EGTA, 25 mM KC1, and 1 mM dithiothreitol) supplemented with [γ-32P] ATP. 2 µl of 6 × SDS sample buffer was added, SDS-PAGE was performed, and then signals were detected by autoradiography (Fig. 12). Phosphorylation of RGC32 by Plk1 was detected (target sequences to be phosphorylated by Plkl were present in Thr82 and Thr113 in RGC32). Furthermore, it was confirmed that phosphorylation of α-casein by Plk1 had been inhibited by the RGC32 protein. Accordingly, it was confirmed that the RGC32 protein binds to Plk1 so as to suppress the kinase activity and to delay Plk1 degradation, thereby causing the extension of the G2/M phase.

### (14) Conclusion

(a) Hemizygously deleted genes in the DNAs of 22 types of glioma-derived cell were screened for by the array CGH method, as reported. Abnormalities were observed in the RGC32 gene at high frequencies, as confirmed by a combination of expression analysis data.
(b) In clinical glioma specimens, a significant inverse correlation between RGC32 gene expression levels and malignancies determined based on the pathological classification of such specimens was observed. The expression levels of the RGC32 gene were observed to have a tendency to decrease in the cases with p53 gene point mutation.
(c) It was revealed that RGC32 gene expression is positively controlled by a p53 gene product.
(d) When cancer cells lacking the RGC32 protein were caused to express the RGC32 protein, anchorage-dependent proliferation ability was decreased and the cell proliferation rate was lowered. Hence, it was revealed that the RGC32 protein has functions of a cancer suppressing gene product.
(e) It was revealed that the RGC32 protein is localized in the centrosome site in the nuclear division phase and is involved in cell cycle control during the G2/M phase upon nuclear division in a manner such that it interacts with Plk1 kinase lower the enzyme activity and thus to delay degradation.

### Effect of the Invention

According to the present invention, an agent for suppressing cancer is provided, which comprises an RGC32 gene (the cancer-suppressing functions of which have been discovered) or an RGC32 protein encoded by the gene. Such drug is very useful from a clinical viewpoint, such as in treatment based on individual cases cancer, cancer prognosis improvement, and the like or in view of basic cancer research. The pathological malignancy of a brain tumor patient can be predicted by measuring the expression level of the messenger RNA of the RGC32 gene, confirming the presence of the RGC32 gene on the genomic DNA, or measuring the amount of the RGC32 protein

## Claims

1. An agent for suppressing cancer comprising an RGC32 gene or a gene homologous thereto, wherein the gene homologous to the RGC32 gene is a gene having a nucleotide sequence that is derived from the nucleotide sequence shown in SEQ ID NO: 1 by deletion, addition, or substitution of 1 to 60 nucleotides and encodes a protein having cancer-suppressing activity, or a gene that is capable of hybridizing to the nucleotide sequence under stringent conditions and encodes a protein having cancer-suppressing activity.

2. The agent for suppressing cancer of claim 1, wherein the gene or the gene homologous thereto is incorporated into a vector.

3. The agent for suppressing cancer of claim 2, wherein the vector is a viral vector or a plasmid vector for expression in animal cells.

4. The agent for suppressing cancer of claim 3, wherein the viral vector is a retrovirus vector, an adenovirus vector, an adeno-associated virus vector, a baculovirus vector, a vaccinia virus vector, or a lentivirus vector.

5. The agent for suppressing cancer of claim 1, wherein the gene or the homologous gene is encapsulated in a liposome.

6. An agent for suppressing cancer which comprises an RGC32 protein as depicted in SEQ ID NO: 2 or a protein homologous thereto having 70% or more homology and having cancer suppressing activity.

## Patentansprüche

1. Mittel zum Unterdrücken von Krebs, umfassend ein RGC32-Gen oder ein dazu homologes Gen, wobei das zum RGC32-Gen homologe Gen ein Gen ist, das eine Nucleotidsequenz hat, die von der Nucleotidsequenz, die in SEQ ID Nr.: 1 gezeigt ist, durch Deletion, Addition oder Substitution von 1 bis 60 Nucleotiden stammt und ein Protein mit krebs-unterdrückender Wirkung kodiert, oder ein Gen, das in der Lage ist mit der Nucleotidsequenz unter stringenten Bedingungen zu hybridisieren und ein Protein mit Krebs-unterdrückender Wirkung kodiert.

2. Mittel zum Unterdrücken von Krebs nach Anspruch 1,
wobei das Gen oder das dazu homologe Gen in einen Vektor eingeschlossen ist.

3. Mittel zum Unterdrücken von Krebs nach Anspruch 2, wobei der Vektor ein viraler Vektor oder ein Plasmidvektor zur Expression in Tierzellen ist.

4. Mittel zum Unterdrücken von Krebs nach Anspruch 3, wobei der virale Vektor ein Retrovirusvektor, ein Adenovirus-Vektor, ein Adeno-assoziiertes Virus-Vektor, ein Baculovirus-Vektor, ein Vacciniavirus-Vektor oder ein Lentivirus-Vektor ist.

5. Mittel zum Unterdrücken von Krebs nach Anspruch 1, wobei das Gen oder das homologe Gen in einem Liposom verkapselt ist.

6. Mittel zum Unterdrücken von Krebs, welches ein RGC32-Protein umfasst, das in SEQ ID Nr.: 2 wiedergegeben ist oder ein Protein, das mit 70 % oder mehr Homologie damit homolog ist und Krebs-unterdrückende Wirkung hat.

## Revendications

1. Agent oncosuppresseur comprenant un gène RGC32 ou un gène homologue, où le gène homologue du gène RGC32 est un gène ayant une séquence nucléotidique dérivée de la séquence nucléotidique présentée dans SEQ ID NO:1 par délétion, addition ou substitution de 1 à 60 nucléotides et qui code une protéine ayant une activité oncosuppressive, ou un gène qui est capable d'hybridation avec la séquence nucléotidique dans des conditions stringentes et qui code une protéine ayant une activité oncosuppressive.

2. Agent oncosuppresseur selon la revendication 1, où le gène ou le gène homologue est incorporé dans un vecteur.

3. Agent oncosuppresseur selon la revendication 2, où le vecteur est un vecteur viral ou un vecteur plasmidique pour expression dans des cellules animales.

4. Agent oncosuppresseur selon la revendication 3, où le vecteur viral est un vecteur de type rétrovirus, un vecteur de type adénovirus, un vecteur de type virus adéno-associé, un vecteur de type baculovirus, un vecteur de type virus de la vaccine ou un vecteur de type lentivirus.

5. Agent oncosuppresseur selon la revendication 1, où le gène ou le gène homologue est encapsulé dans un liposome.

6. Agent oncosuppresseur qui comprend une protéine RGC32 telle que décrite dans SEQ ID NO:2 ou une protéine homologue ayant une homologie de 70 % ou plus et ayant une activité oncosuppressive.


### SEQUENCE LISTING

<110> FUJIFILM Corporation
<120> A cancer-suppressing agent
<130> FA7142A
<160> 2
<210> 1
   <211> 895
   <212> DNA
   <213> Homo sapiens
<400>
   gcggccgcgt cgaccggcgc ggctggagcg cagcgccgaa gggactggca gggctgaagt 60
   gtgcgggaca gcaagccccc gaatagcccc ggctgccacc tcgcaggacc caaggccacg 120
   cgcgccgggc ccagctgagc cgcctcatga agccgcccgc ggaggacctg tcggacgcgc 180
   tgtgcgagtt tgacgcggtg ctggccgact tcgcgtcgcc cttccacgag cgccacttcc 240
   actacgagga gcacctggag cgcatgaagc ggcgcagcag cgccagtgtc agcgacagca 300
   gcggcttcag cgactcggag agtgcagatt cactttatag gaacagcttc agcttcagtg 360
   atgaaaaact gaattctcca acagactcta ccccagctct tctctctgcc actgtcactc 420
   ctcagaaagc taaattagga gacacaaaag agctagaagc cttcattgct gatcttgaca 480
   aaactttagc aagtatgtga aacaagaagt tctgggtcct ttcatcataa gggagaagct 540
   tcagaaagtt ccgaggacct gctaaaatca gctactagaa tctgctgcca gaggggacaa 600
   agacgtgcac tcaaccttct accaggccac tctcaggctc accttaaaat cagcccttga 660
   tcccatttct gggcaattta gacagtgaaa ctgactttgt ttacctgctt gcagcatatt 720
   agaacagacg atccatgcta atattgtatt ttctcttaaa acatagcttt cctgtaattt 780
   aaagtgcttt tatgaaaata tttgtaatta attatatata gttggaaata gcagtaagct 840
   ttcccattat aatatatttt tgtatacaaa taaaatttga actgaacctc gtgcc      895
<210> 2
   <211> 117
   <212> PRT
   <213> Homo sapiens
<400> 2
   Met Lys Pro Pro Ala Glu Asp Leu Ser Asp Ala Leu Cys Glu Phe Asp
   1 5 10 15
   Ala Val Leu Ala Asp Phe Ala Ser Pro Phe His Glu Arg His Phe His
   20 25 30
   Tyr Glu Glu His Leu Glu Arg Met Lys Arg Arg Ser Ser Ala Ser Val
   35 40 45
   Ser Asp Ser Ser Gly Phe Ser Asp Ser Glu Ser Ala Asp Ser Leu Tyr
   50 55 60
   Arg Asn Ser Phe Ser Phe Ser Asp Glu Lys Leu Asn Ser Pro Thr Asp
   65 70 75 80
   Ser Thr Pro Ala Leu Leu Ser Ala Thr Val Thr Pro Gln Lys Ala Lys
   85 90 95
   Leu Gly Asp Thr Lys Glu Leu Glu Ala Phe Ile Ala Asp Leu Asp Lys
   100 105 110
   Thr Leu Ala Ser Met
   115